# EUROPEAN PATENT APPLICATION

(11) **EP 2 361 602 A2**
(43) Date of publication of application: **31.08.2011**
(21) Application number: 11154255.1
(22) Date of filing: 11.02.2011
(51) Int. Cl.: A61K 8/04, A61Q 5/00

(54) **A hair building solid agent**

(30) Priority: 11.02.2010 GB 1002254
(71) Applicant: Pangaea Laboratories Limited, London NW2 2AD (GB)
(72) Inventor: Isaacs, Elliot, London, NW2 2AD (GB)
(74) Representative: Hands, Lewis Roger

(57) **Abstract**

A method of treating hair comprising applying a hair building solid agent (30) for use on or with one or more of hair (10), skin and hair building solids (20) for altering and/or maintaining the electrostatic charge of the hair and/or skin such that it has a substantially negative polarity. The method further comprises applying hair building solids (20) to hair (10) before and/or during and/or after applying the hair building solid agent (30). The hair building solid agent (30) is applied as a spray on to the hair and/or skin and/or hair building solid.

## Description

The present invention relates generally to a method of treating hair with a hair building solid agent and finds particular, although not exclusive, utility in the improvement of the cosmetic effect of the use of hair building solids.

Hair building solids may take different forms. However, typically they are powders and/or fibres. The fibres are relatively narrow (typically less than 200 nm in diameter) and short (typically less than 0.7 mm in length). They typically comprise keratin, rayon or nylon. The solids may be coloured to match a user's hair. The solids are applied to the scalp and/or the hairs of the user typically in an area where the user has suffered hair loss. The solids adhere to the hairs and/or scalp and in the case of the former help to thicken the hairs' appearance, and in the case of the latter to camouflage the skin.

The adherence of the solids to the scalp/hairs is typically enabled by means of electrostatic charge, although other means such as adhesives (for example hair spray) may be used. The solids are typically electrostatically non-conductive and thus an electrostatic charge may be built up on their surface. This build up may be due to contact with other surfaces, such as other solids, or the container in which the solids are stored. The built-up charge may last a relatively long time on the surface of the solids until such time as they are discharged to a neutral or oppositely charged surface.

In a known embodiment, the solids are provided in a dispenser which includes a sieve at one end in a similar style to a pepper pot. As the solids are dispensed from the dispenser, by either being shaken out, and/or by gravity, the electrostatic charge on the surface of at least some of the solids is affected such that they naturally adhere to the hair and/or scalp of the user. The charge is affected by the movement of the solids across and past the material making up the sieve, and collision with each other. The charge may be affected by the number, size, shape, concavity, angle, spacing, and pattern of the holes in the sieve, and the dimensions of the sieve itself. The material comprising or at least partially coating the sieve and/or the solids may also affect the charging of the solids.

It is known to apply an agent to thicken individual hair shafts in advance of applying hair building solids. The thickening step has two purposes. First, it may alter the appearance of someone's hair in that if it is thinning the thickened hairs at least partly conceal this fact by presenting a fuller head of hair. Second, a thickened hair shaft presents a larger surface area for the hair building solids to adhere to and thus provides better adhesion.

However, this known method has inherent problems. For instance, thickening agents (or cosmetics) such as humectants and conditioners often coat the hair leaving it appearing greasy and heavy. This can cause thinning hair to look thinner. Another problem is that conditioners affect the way hair building solids adhere to hair by means of electrostatic charge since they affect the charge of hair.

This is important because it is well understood that the drier the hair and the less conditioner present the better the adhesion of hair building solids to hair shafts. This is partly because dry hair typically has a charge of a certain amplitude and polarity, and by contrast, well moisturised hair coated with a conditioning agent typically has a charge of a significantly different amplitude and/or polarity. The difference in charge potential is caused by the conditioning agents which accumulates on hair shafts.

Another reason why hair having conditioner present provides a less favourable environment for the use of hair building solids is that individual dry hairs tend not to aggregate into bunches but are discrete from one another. By contrast, moisturised/conditioned hairs tends to lie flat or bunch together, preventing hair building solids from being distributed evenly across a region of hair.

However, it is understood that conditioners are appealing to consumers because of the affect that they have on hair in other ways, such as a smoother, thicker-looking hair, as opposed to un-conditioned, possibly dry, hair with split-ends.

In a first aspect, the invention provides a method of treating hair comprising the steps of:
1. Providing a hair building solid agent for use on, or with, one or more of hair, skin and hair building solids for altering and/or maintaining the electrostatic charge of the hair and/or skin such that it has a substantially negative polarity;
2. Applying said hair building solid agent to one or more of hair, skin and hair building solids;
3. Providing hair building solids; and
4. applying hair building solids to hair before and/or during and/or after applying the hair building solid agent;

characterised in that the hair building solid agent is applied as a spray on to one or more of the hair, skin and hair building solid.

The method may be arranged such that a majority of the hair building solids have one end electrostatically adhered to the hairs and the opposite longitudinal end located away from the hairs. In this way, the hair is provided with more "body" or "thickened". In this regard, the term "majority" may be replaced by the term "substantial number".

In a second aspect, the invention provides a hair building solid agent spray for use on or with one or more of hair, skin and hair building solids for altering and/or maintaining the electrostatic charge of the hair and/or skin such that it has a substantially negative polarity.

In this respect, it is understood that clean dry hair may naturally have a negative charge. This may also apply to skin. Hair building solids may have a generally overall negative charge (for instance at both ends of each solid) but on application of the solids to hair it is understood that at least a portion of each solid may become positively charged. This may be at one end of each solid. This allows the solids to adhere electrostatically to negatively charged hair. For optimum hair thickening the solids adhere at one end to the hair, the other end being located substantially away from the hair, so as to "thicken" the look of the hair. However, if typical, well-known conditioner is applied to hair the conditioner coats the hairs such that the conditioner coating exhibits a generally positive charge. This tends to result in hair building solids either not adhering to the hairs or adhering such that they lie flat against the hair so that the hair is not "thickened". By contrast, if the hair building solid spray agent is applied to the hair such that it coats them then the coating may exhibit a generally negative charge. This allows for the solids to adhere to the hair at one end such that the hair appears thickened while maintaining at least some of the benefits of conditioned hair. The hair building solid spray agent may be applied before, during or after application of the hair building solids.

The spray agent may comprise one or more naturally amphoteric substance, such as a surfactant, a conditioner, cocamidopropylbetaine, dodecylbetaine, cocoamphoglycinate, oleyl betaine, soyamidopropylbetaine and an amino acid. The amino acid may be dipolar. Alternatively, or additionally, the amino acid may be one or more of a soy amino acid and a hydrolysed wheat protein amino acid.

Amphoteric molecules may be used. Amphoteric surfactants and dipolar amino acids may both coat the hair and may have conditioning properties. Also, dipolar amino acids may swell and thicken hair shafts.

An effect of using such molecules or substances is that they may bind to a damaged negatively charged hair in such a fashion that the positive part of the molecules may be in contact with the hair, and the negative part may form the surface of the coating. Therefore, when a hair building solid is applied, the surface charge may be the same as that of dry hair and so binding may be improved.

The hair building solid spray agent may include a water-resistant agent. This may at least partly waterproof the hair, or the hair and hair building solid together, after the solid is applied. As discussed earlier, water content may have a significant effect on electrostatic charge. Accordingly, it may be important to maintain the optimum moisture content in hair and/or hair building solids such that an optimum electrostatic charge potential for enhancing the adhesion of hair building solids to hair and/or skin is effected.

Coating the hair or hair/hair building solid combination with an alcohol or oil soluble polymer may maintain the amount of water/moisture in the hair and the hair building solid, thus possibly maintaining the optimum charge potential for effective adhesion/binding. Accordingly, the hair building solid spray agent may include an alcohol and/or oil soluble polymer.

The substance may be one or more of octylacrylamide copolymer, C12-22 alkylmethacrylate copolymer, PVP/dimethylaminoethylmethacrylate copolymer, polyquaternium 69, isobutylene ethylmaleimide copolymer, isobutylene hydroxyethylmaleimide copolymer, polymethylsilsesquioxane and polyurethane-14 AMP-Acrylates copolymer.

The hair building solid spray agent may include an humectant and/or an emollient. The humectant and/or emollient may be one or more of a hyaluronate, panthenol, glycerin and cocos nucifera oil.

Coconut oils, such as cocos nucifera oil, may act as highly penetrative emollients. These may have the effect of swelling hair shafts to the same extent as other emollients but by penetrating and swelling the shaft from within, without significantly coating the hair shaft. This may avoid an excessive build-up of emollient coating hair, allowing the use of amphoteric hair thickening agents without undue overall quantities of agents on the hair surface.

The hair building solid spray agent may include an antioxidant.

The antioxidant may be one or more of a superoxide dismutase, a superoxide dismutase mimetic, catalase mimetics, vitamin C and its derivatives, alpha lipoic acid, retinoic acid, vitamin A and its derivatives, copper PCA, dimethylmethoxy chromanol and a form of vitamin E and its derivatives.

The hair building solid spray agent may include an amino acid that is non-dipolar.

The non-dipolar amino acid may be one or more of L-cysteine, L-pyrrolidone, histadine, methionine, glycine, lysine tryptophan, glutamate and glutamine. Alternatively, or additionally, the non-dipolar amino acid may be in the form of a peptide. The peptide may be a dipeptide or a tripeptide. The non-dipolar amino acid and/or peptide may be complexed or chelated with, or a salt of, copper, zinc, manganese, iron or any other 2+ valency transition metal ions.

The hair building solid spray agent may include one or more pharmacologically active ingredient for treating one or more of hair loss, thinning hair and skin conditions.

The pharmacologically active ingredient may be one or more of finasteride, dutasteride, spironolactone, minoxidil, nitric oxide donators, β-glucan, saw palmetto, resveratrol, curcumin, marine extracts, polycyanidins, superoxide dismutase, superoxide dismutase mimetics, taurine, plant sterols, pine bark extract, melatonin, green tea, caffeine, copper peptides, copper PCA, EUK-134, copper(II) 3,5-disopropylsalicylate, dimethylmethoxy chromanol, catalase, catalase mimetics and hydrolysed lupine protein.

The hair building solid spray agent may include one or more UV resistant ingredients. UV resistant ingredients may protect the other ingredients and/or the hair and/or the hair building solid agent from the effects of UV. UV resistant ingredients may preserve the optimum water content and/or colour of the hair and/or hair building solid.

The UV resistant ingredient may be one or more of benzophenone-3, benzophenone-4, titanium oxide, aluminium oxide, quaternium-95 and propenediol.

The hair building solid spray agent may include a hair building solid. The hair building solid may comprise a coating permitting the solid to charge in a bipolar fashion.

The hair building solid spray agent may include a propellant in the form of a pressurised gas or liquid.

The hair building solid agent may be for use with a component of a hair styling or treatment device. The treatment device may comprise one or more of a comb, brush, conductive element, fan and a hairdryer.

In another embodiment, the hair building solid agent is not in the form of a spray and thus the methods described herein use a liquid, powder or other such non-spray like formulation. However, all other features described herein relating to the methods using a spray may also relate to this non-spray method.

These methods may be for altering and/or maintaining the electrostatic charge of the hair, skin and/or hair building solids such that it has a substantially negative polarity.

The methods may include the step of providing a device for maintaining and/or altering the electrostatic charge of the hair, skin, or hair building solid.

In one embodiment, the present invention is concerned with cosmetic formulations that allow conditioning, moisturising, thickening and/or cleaning of a hair shaft and simultaneously provide the correct electrostatic conditions for the binding of a hair building solid to said hair shaft.

In a third aspect, the invention provides the use of a hair building solid agent as a hair building solid electrostatic binding agent. The hair building solid agent may alter and/or maintain the electrostatic charge of hair and/or skin to have a substantially negative polarity, and the hair building solids may be arranged such that a majority of them have one end electrostatically attracted to the hairs and/or skin and the opposite longitudinal end located away from the hairs and/or skin.

The hair building solid agent, and its use, may have any one or more of the characteristics described and/or claimed herein with respect to any of the other aspects or embodiments.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

Figure 1 shows dry hair with hair building solids;

Figure 2 shows hair treated with a known conditioner with hair building solids;

Figure 3 shows a portion of a hair including the electrostatic charges relating to Figure 1; and

Figure 4 shows a portion of a hair including an agent according to one embodiment of the invention with hair building solids.

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn to scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may refer to different embodiments. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those skilled in the art. For example, in the following claims, any of the claimed embodiments can be used in any combination.

In the description provided herein, numerous specific details are set forth. However, it is understood that embodiments of the invention may be practised without these specific details. In other instances, well-known methods, structures and techniques have not been shown in detail in order not to obscure an understanding of this description.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

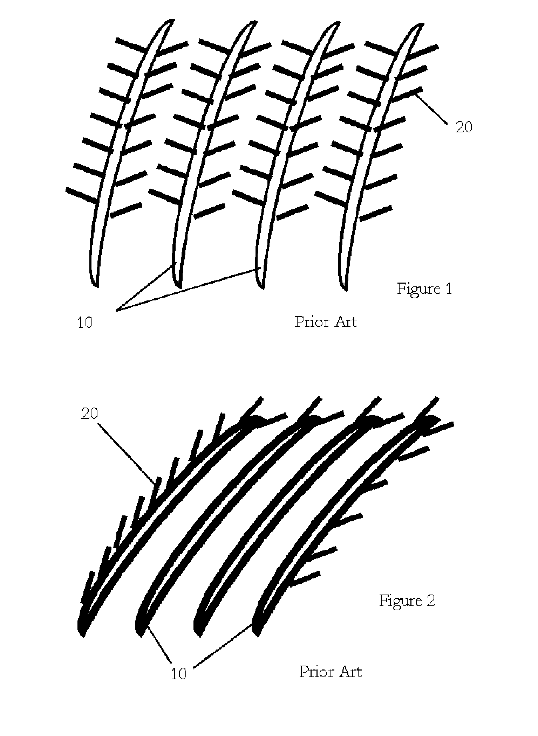

In Figure 1, hair building solids 20 have been applied to dry hair 10 and are shown adhered at one end of their fibre-like shape to the hairs 10 with their other opposite ends extending away. In this manner, hair 10 may have a "thickened" appearance.

By contrast, in Figure 2, the hair 10 includes a known conditioner which results in the hair clumping together and/or the poor adhesion of the hair building solids 20 thereto. Furthermore, the hair building solids 20 may lie closer to the longitudinal lengths of each hair 10 such that they extend away from the hair at a reduced angle. In this manner, the hair 10 has a less "thickened" appearance of that shown in Figure 1.

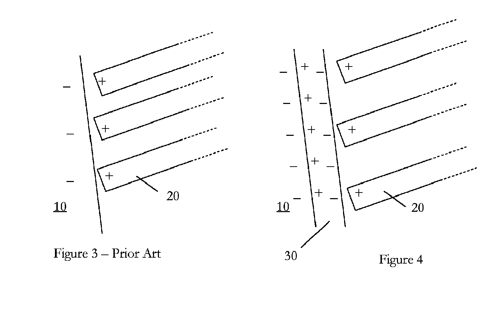

Figure 3 shows a portion of a dry hair. The hair's outer wall is indicated by the approximately vertical line. The polarity of the electrostatic charge of the hair 10 is generally negative, as indicated. Hair building solids 20 have at least one portion which is generally positive and are thus attracted to, and remain in place adjacent, the hair 10.

Figure 4 shows the hair 10 of Figure 3 but including a coating of hair building solid agent 30. This coating 30 has a generally positive inner portion, which is attracted/adheres to the generally negative hair 10, and a generally negative outer portion. This generally negative portion attracts the generally positive portion of the hair building solids 20 such that the solids 20 are substantially retained in place such that one end of the solid is adjacent the hair 10 and the other end extends away therefrom. In this manner, hair 10 may have the preferred appearance of having been conditioned but may also appear "thickened" by means of the hair building solids.

Although not shown in Figure 4 it is to be understood that the hair building solids 20 may be attracted to the hair 10 at any point on its surface and not just on one side.

## Claims

1. A method of treating hair comprising the steps of: a) providing a hair building solid agent for use on, or with, one or more of hair, skin and hair building solids for altering and/or maintaining the electrostatic charge of the hair and/or skin such that it has a substantially negative polarity; b) applying said hair building solid agent (30) to one or more of hair (10), skin and hair building solids (20); c) providing hair building solids; and d) applying hair building solids (20) to hair (10) before and/or during and/or after applying the hair building solid agent (30); **characterised in that** the hair building solid agent (30) is applied as a spray on to one more of the hair (10), skin and hair building solid (20).

2. The method of claim 1, being arranged such that a majority of the hair building solids (20) have one end electrostatically adhered to the hairs (10) and the opposite longitudinal end located away from the hairs.

3. The method of either one of claims 1 and 2, in which the hair building solid agent (30) comprises one or more naturally amphoteric substance from the following: a surfactant, a conditioner, cocamidopropylbetaine, dodecylbetaine, cocoamphoglycinate, oleyl betaine, soyamidopropylbetaine and an amino acid.

4. The method of claim 3 wherein the hair building solid agent (30) is a dipolar amino acid.

5. The method of any preceding claim, in which the hair building solid agent (30) includes a water-resistant agent.

6. The method of claim 5, wherein the water resistant agent is one or more of octylacrylamide copolymer, C12-22 alkylmethacrylate copolymer, PVP/dimethylaminoethylmethacrylate copolymer, polyquaternium 69, isobutylene ethylmaleimide copolymer, isobutylene hydroxyethylmaleimide copolymer, polymethylsilsesquioxane and polyurethane-14 AMP-Acrylates copolymer.

7. The method of any preceding claim, in which the hair building solid agent (30) includes an antioxidant being one or more of a superoxide dismutase, a superoxide dismutase mimetic, catalase mimetics, vitamin C and its derivatives, alpha lipoic acid, retinoic acid, vitamin A and its derivatives, copper PCA, dimethylmethoxy chromanol and a form of vitamin E and its derivatives.

8. The method of any preceding claim, in which the hair building solid agent (30) includes an amino acid that is non-dipolar.

9. The method of claim 8, wherein the amino acid is one or more of L-cysteine, L-pyrrolidone, histadine, methionine, glycine, lysine tryptophan, glutamate and glutamine.

10. The method of any preceding claim, in which the hair building solid agent (30) includes one or more pharmacologically active ingredient for treating one or more of hair loss, thinning hair and skin conditions.

11. The method of claim 10, wherein the pharmacologically active ingredient is one or more of finasteride, dutasteride, spironolactone, minoxidil, nitric oxide donators, β-glucan, saw palmetto, resveratrol, curcumin, marine extracts, polycyanidins, superoxide dismutase, superoxide dismutase mimetics, taurine, plant sterols, pine bark extract, melatonin, green tea, caffeine, copper peptides, copper PCA, EUK-134, copper(II) 3,5-disopropylsalicylate, dimethylmethoxy chromanol, catalase, catalase mimetics and hydrolysed lupine protein.

12. The method of any preceding claim, in which the hair building solid agent (30) includes one or more UV resistant ingredients being one or more of benzophenone-3, benzophenone-4, titanium oxide, aluminium oxide, quaternium-95 and propenediol.

13. A hair building solid agent spray for use on or with one or more of hair, skin and hair building solids for altering and/or maintaining the electrostatic charge of the hair and/or skin such that it has a substantially negative polarity.

14. Use of a hair building solid agent as a hair building solid electrostatic binding agent.

15. The use of claim 14, wherein the hair building solid agent alters and/or maintains the electrostatic charge of hair and/or skin to have a substantially negative polarity, and the hair building solids are arranged such that a majority of them (20) have one end electrostatically attracted to the hairs (10) and/or skin and the opposite longitudinal end located away from the hairs and/or skin.
